# EUROPEAN PATENT APPLICATION

(11) **EP 4 772 165 A1**
(43) Date of publication of application: **08.07.2026**
(21) Application number: 24859766.8
(22) Date of filing: 27.08.2024
(51) Int. Cl.: A61K 8/73, A61K 8/81, A61Q 1/10

(54) **AQUEOUS LIQUID COSMETIC**

(30) Priority: 01.09.2023 JP 2023142165
(71) Applicant: MITSUBISHI PENCIL COMPANY, LIMITED, Tokyo 140-8537 (JP)
(72) Inventor: YOSHIMURA Ai, Fujioka-shi, Gunma 375-8501 (JP); YAMAZAKI Yuichi, Fujioka-shi, Gunma 375-8501 (JP)
(74) Representative: Vossius & Partner Patentanwälte Rechtsanwälte mbB
(86) International application number: PCT/JP2024/030485
(87) International publication number: WO 2025/047734

(57) **Abstract**

Provided is an aqueous liquid cosmetic which can be suitably loaded onto an applicator including an application portion having a brush, such as a direct liquid type eyeliner, an eyeshadow, and an eyebrow product for drawing a line on eyebrows, by suppressing sedimentation of a plate-shaped pigment exhibiting glittering properties, and which enables drawing or the like of a line having a constant hue and sparkling effect over time. The aqueous liquid cosmetic of the present disclosure is characterized by containing at least (a) 0.01 to 10 mass% of a plate-shaped pigment having a surface coated with a compound selected from Group A, (b) at least one chromatic pigment having an average particle size of 0.01 to 1 µm as measured by dynamic light scattering, (c) 1 to 20 mass% of an acrylic copolymer in terms of solid content, and (d) water, the aqueous liquid cosmetic being loaded onto an applicator having an application portion formed of a brush: Group A: cellulose, hemicellulose, lignin, chitin, and chitosan.

## Description

### Technical Field

The present specification relates to an aqueous liquid cosmetic containing a plate-shaped pigment exhibiting glittering properties, and more particularly, to an aqueous liquid cosmetic which can be suitably used for an applicator such as a direct liquid type eyeliner, an eyeshadow, and an eyebrow product for drawing a line on eyebrows by suppressing sedimentation of the plate-shaped pigment.

### Background Art

In the related art, an aqueous liquid cosmetic for forming a glittering coating film on an application surface, such as skin or eyebrows, generally contains a plate-shaped pigment (glitter pigment) exhibiting glittering properties.
However, the plate-shaped pigment is liable to sediment, and once it sediments to form a hard cake in a layer form, the layered structure is difficult to peel off again and difficult to redisperse in a liquid. Therefore, the glitter pigments overlap even in the drawn line and fail to exhibit the sparkling effect corresponding to the blending amount.

A known method for solving the above problem is, for example, to prevent the formation of a hard cake of a layered plate-shaped pigment by increasing the viscosity of a vehicle, but this technique has a problem in that a discharge means and a structure thereof are limited.

On the other hand, reference may be made to patent documents relating to aqueous liquid cosmetics containing plate-shaped pigments and applicators thereof, and examples thereof include:
1) an aqueous cosmetic containing (a) a glitter pigment having an average particle size of 10 to 100 µm, (b) an acrylic resin emulsion, and (c) a thickener selected from the group consisting of silica, clay minerals, palmitoyl dipeptide, cellulose, cellulose nanofibers, and xanthan gums, in which a content of the component (c) is 0.1 to 0.35 mass%, and a viscosity at 25°C is 5 to 30 mPa·s, for the purpose of providing an aqueous cosmetic that has high glittering properties and can be used as a brush-pen type eyeliner (for example, see Patent Document 1).
2) An aqueous eyeliner composition containing a combination of a glitter pigment, carbon black, a microorganism-derived polysaccharide, a volatile alcohol, and an alkyl acrylate copolymer emulsion, to provide an aqueous eyeliner composition having excellent abrasion resistance, vivid color development, smooth writing feeling, and good storage stability (for example, see Patent Document 2).
3) An aqueous makeup cosmetic containing (A) an aqueous alkali-thickened polymer emulsion, (B) a pigment, (C) a glittering powder, (D) an ethanol-soluble film forming agent, and (E) an alkali, for the purpose of providing an aqueous makeup cosmetic that spreads smoothly and has excellent color development and glossiness, a high cosmetic effect, good adhesion to the skin, excellent cosmetic durability, and excellent storage stability, the aqueous makeup cosmetic containing at least carbon black as the component (B) (for example, see Patent Document 3).
4) There are known liquid cosmetic composition containing 0.01 to 10 mass% of a plate-shaped pigment having a surface coated with at least a compound selected from the group consisting of cellulose, hemicellulose, lignin, chitin, and chitosan, 0.05 to 5 mass% of layered clay mineral particles, 1 to 20 mass% of an acrylic copolymer in terms of solid content, and water, the liquid cosmetic composition characterized by being filled in an applicator equipped with an application portion having a brush, and the like, for the purpose of providing a liquid cosmetic composition that easily provides a clear glittering coating film without overlapping of plate-shaped pigments even when the liquid cosmetic composition containing a small amount of the plate-shaped pigment having glittering properties is filled in an applicator having an application portion such as a brush and applied onto an application surface such as skin (for example, see Patent Document 4).

However, in the aqueous liquid cosmetics containing the plate-shaped pigments of Patent Documents 1 to 4, there is still a problem that the sparkling effect of the plate-shaped pigments is impaired when pigments, layered viscous minerals, and the like are blended to improve the color and the like. Moreover, when plate-shaped pigments remain overlapped on the drawn line, there is a problem such as a decrease in sparkling effect, and further, the plate-shaped pigments form a structure with another blended pigment, resulting in a layered hard cake that makes redispersion in the liquid difficult. This hinders the discharge of the pigment and causes a problem that the color development and the sparkling effect corresponding to the blending amount cannot be exhibited. Furthermore, when the aqueous liquid cosmetics of Patent Documents 1 to 3 are loaded (filled) onto a direct liquid type eyeliner, an eyeshadow, and the like including an application portion having a brush, there are still problems such as insufficient discharge and poor drawing properties (application properties).

### Citation List

### Patent Document

Patent Document 1: JP 2022-131500 A (Claims, Examples, etc.)
Patent Document 2: JP 2007-153744 A (Claims, Examples, etc.)
Patent Document 3: JP 2017-114803 A (Claims, Examples, etc.)
Patent Document 4: JP 2021-14412 A (Claims, Examples, etc.)

### Summary of Invention

### Technical Problem

The present disclosure has been made in view of the above known problems and the current situation, and aims to solve the problems, and an object thereof is to provide an aqueous liquid cosmetic which can be suitably loaded onto an applicator such as a direct liquid type eyeliner, an eyeshadow, and an eyebrow product for drawing a line on eyebrows, which are provided with an application portion having a brush, by suppressing sedimentation of a plate-shaped pigment exhibiting glittering properties. Also it enables a drawing line to keep a hue and a sparkling effect constant over time.

### Solution to Problem

As a result of intensive studies on the above-described known problems and the like, the inventors of the present disclosure have found that an aqueous liquid cosmetic of the above-described object can be obtained by incorporating at least a predetermined amount of a plate-shaped pigment having a surface coated with a compound having specific physical properties, a chromatic pigment having a particle size in a specific range, and a predetermined amount of an acrylic copolymer and water, and loading the resultant in an applicator including application portion formed of a brush, and have completed the present disclosure.

That is, the aqueous liquid cosmetic of the present disclosure is characterized by containing at least (a) 0.01 to 10 mass% of a plate-shaped pigment having a surface coated with a compound selected from Group A, (b) at least one chromatic pigment having an average particle size of 0.01 to 1 µm as measured by dynamic light scattering, (c) 1 to 20 mass% in terms of solid content of an acrylic copolymer, and (d) water, the aqueous liquid cosmetic being loaded onto an applicator having an application portion formed of a brush:
Group A: cellulose, hemicellulose, lignin, chitin, and chitosan. The aqueous liquid cosmetic preferably further contains a thickener.
The thickener is preferably a dextrin and/or a thickening polysaccharide.
The aqueous liquid cosmetic preferably has a viscosity of 15.0 mPa·s or less as measured at 25°C and at a shear rate of 191.5 s⁻¹.

### Advantageous Effects of Invention

According to the present disclosure, there is provided an aqueous liquid cosmetic which can be suitably loaded onto an applicator including an application portion having a brush, such as a direct liquid type eyeliner, an eyeshadow, and an eyebrow product for drawing a line on eyebrows, by suppressing sedimentation of a plate-shaped pigment exhibiting glittering properties, and which enables drawing of a line to have a hue and a sparkling effect constant over time.
The object and effects of the present disclosure can be recognized and obtained especially using the components and combinations indicated in the claims. Both general explanation described above and detailed explanation described below are exemplary and explanatory and do not limit the present disclosure described in claims.

### Brief Description of Drawings

FIGS. 1(a) and 1(b) are a perspective view and a longitudinal sectional view, respectively, showing an example of an embodiment of an applicator to be filled with an aqueous liquid cosmetic of the present disclosure, the applicator having an application portion formed of a brush.
FIGS. 2(a) and 2(b) are drawings showing an example of a pen-type application portion used in Comparative Examples, in which FIG. 2(a) is a front view and FIG. 2(b) is a sectional view taken along line X-X of FIG. 2(a).

### Description of Embodiments

Embodiments of the present disclosure will be described below in detail. However, it should be noted that the technical scope of the present disclosure is not limited to the embodiments detailed below and includes the invention described in claims and equivalents thereof. The present disclosure can be implemented based on the contents disclosed in the present specification and technical common knowledge (including design matters and obvious matters) in the art.
The aqueous liquid cosmetic of the present disclosure is characterized by containing at least (a) 0.01 to 10 mass% of a plate-shaped pigment having a surface coated with a compound selected from Group A, (b) at least one chromatic pigment having an average particle size of 0.01 to 1 µm as measured by dynamic light scattering, (c) 1 to 20 mass% of an acrylic copolymer in terms of solid content, and (d) water, the aqueous liquid cosmetic being loaded onto an applicator having an application portion formed of a brush:
Group A: cellulose, hemicellulose, lignin, chitin, and chitosan.

The plate-shaped pigment used in the present disclosure has glittering properties and is a plate-shaped pigment whose surface is coated with at least a compound selected from Group A. The examples thereof include a plate-shaped pigment such as a pearl pigment, an aluminum flake pigment (aluminum powder pigment), a metal- or metal oxide-coated glass flake, and an aluminum-coated polyester film, whose surface is coated with at least a compound selected from Group A.

In the present disclosure, at least one compound selected from cellulose polymers (oxidized cellulose, fermented cellulose and the like), hemicellulose, lignin, chitin, and chitosan (each alone or in combination of two or more, the same applies hereinafter) can be used as the compound selected from Group A.
Examples of the method for coating the surface of the plate-shaped pigment with the compound include 1) a method of adhering at least one compound selected from Group A to the surface of the plate-shaped pigment by spraying and drying a water slurry mixture of the compound selected from Group A and the plate-shaped pigment using a spray dryer (hereinafter referred to as "Production Method 1"), and 2) a method of adjusting the pH of a substance soluble in an acidic or basic aqueous solution, dissolving the soluble substance in water, shifting the pH in the direction of insolubilization with an acid or base, and precipitating at least one compound selected from Group A on the surface of the plate-shaped pigment (hereinafter referred to as "Production Method 2"). When the surface coating amount of the compound selected from Group A is too small, the effect of redispersibility by the suppression of hard cake is not obtained, and on the other hand, when it is too large, not only the pearl feeling (glittering property) is reduced, but also the pearl surface becomes not a point contact but a surface contact, and the effect of redispersibility is not obtained. Therefore, it is desirable that the surface coating amount of the compound selected from Group A is in the range of 0.1 to 10%, preferably 0.5 to 8%, and more preferably 1 to 7%, as a surface coating ratio of the plate-shaped pigment (calculated from mass% at the time of surface treatment).

Specifically, as the plate-shaped pigment to be used, for example, a commercially available product obtained by coating a surface of a plate-shaped pigment such as aluminum flake pigment, metal oxide-coated glass flake (trade name "Metashine" or the like), or metal oxide-coated mica (trade name "Lumina" or the like) with a compound selected from Group A, or a commercially available product of a plate-shaped pigment having a surface formed of a compound selected from Group A can be used.
As the particle size of the smooth surface of the coated plate-shaped pigment to be used is larger, the sparkling effect is generally higher, but it is preferably about 5 µm to 100 µm from the viewpoint of suitably exhibiting the effect of the present disclosure.
In the present disclosure (including Examples), the "particle size" or "average particle size" described later refers to a value measured and calculated by a dynamic light scattering method [particle size analyzer FPAR-1000, available from Otsuka Electronics Co., Ltd.], or a value of D50 calculated on a volume basis using a particle size distribution analyzer HR9320-X100 (available from Nikkiso Co., Ltd.).
The plate-shaped pigment coated with a compound selected from Group A obtained as described above and used in the present disclosure can be confirmed to have crystals of the compound on the surface when observed with an electron microscope (×35000 times).
In addition, in the present disclosure, as a method for distinguishing between a plate-shaped pigment having a surface coated with a compound selected from Group A and an uncoated plate-shaped pigment, it can be confirmed that redispersibility is not obtained in a system in which an uncoated plate-shaped pigment is blended, but good redispersibility is obtained in a system in which a compound selected from Group A is used for surface coating of plate-shaped particles.

In the present disclosure, desirably, the content of the plate-shaped pigment coated with the compound selected from Group A is preferably 0.01 to 10 mass% (hereinafter, "mass%" is simply referred to as "%"), more preferably 0.5 to 5%, with respect to the total amount of the aqueous liquid cosmetic, for obtaining sufficient glittering properties and concealing power when the product is used.
When the content of the surface-treated plate-shaped pigment is less than 0.01%, the glittering properties become insufficient. Whereas, when the content is more than 10%, the high concentration of the plate-shaped pigment inevitably increases the sparkling effect and also raises the cost, thereby diminishing the significance of the present disclosure, and further, upon application, application properties tend to deteriorate or application defects are likely to occur, which is undesirable. Note that in the case of a plate-shaped pigment which is not surface-treated, the formation of a hard cake is accelerated.

The component (b) used in the present disclosure is at least one chromatic pigment having an average particle size of 0.01 to 1 µm as measured by dynamic light scattering, preferably a chromatic pigment of 0.05 µm to 0.8 µm. When the average particle size of the chromatic pigment is less than 0.01 µm, deterioration in redispersibility due to the formation of hard cake is observed, whereas when the average particle size is more than 1 µm, deterioration in vertical hue difference due to sedimentation of the chromatic pigment is observed.
As the chromatic pigment that can be used, both an inorganic color pigment and an organic color pigment are used. The shape and particle structure of these chromatic pigments are not particularly limited as long as they are generally used in the field of cosmetics.

Examples of the inorganic color pigment include inorganic white pigments such as titanium oxide and zinc oxide; inorganic red-based pigments such as iron oxide, iron hydroxide, and iron titanate; γ-Inorganic brown pigments such as iron oxide; inorganic yellow pigments such as yellow iron oxide and yellow earth; inorganic black pigments such as black iron oxide and carbon black; inorganic violet pigments such as manganese violet and cobalt violet; inorganic green pigments such as chromium hydroxide, chromium oxide, cobalt oxide, and cobalt titanate; inorganic blue pigments such as Prussian blue and ultramarine blue.
Examples of the organic color pigment include Red No. 201, Red No. 202, Red No. 204, Red No. 205, Red No. 220, Red No. 226, Red No. 228, Red No. 405, Orange No. 203, Yellow No. 205, Yellow No. 4, Yellow No. 5, Blue No. 1, and Blue No. 404; lakes (zirconium lake, barium lake, aluminum lake, and the like) of a water-soluble dye such as Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1; and natural pigments such as chlorophyll and β-carotene and lakes thereof.

The chromatic pigments having a predetermined particle size may be used alone, or two or more thereof may be used in combination. The total content of the chromatic pigments having a predetermined particle size can be appropriately selected depending on the use of the aqueous liquid cosmetic. The content is preferably 0.01 to 15%, more preferably 0.05 to 10% with respect to the total amount of the aqueous liquid cosmetic.
When the content of the chromatic pigments is excessively large exceeding 15%, it is difficult to discharge the liquid, and conversely, when the content is less than 0.01%, the effect of the coloring material may be insufficient.

The acrylic copolymer as the component (c) used in the present disclosure is used from the viewpoint of further fixability and dispersion stability. Examples thereof include those containing at least one of an alkyl acrylate copolymer, an acrylate copolymer, ammonium acrylate copolymer, an acrylic resin alkanolamine liquid, an alkyl acrylate-vinyl acetate copolymer, an (alkyl acrylate/octylacrylamide) copolymer, a silicone-modified acrylic copolymer, and an octylamide-acrylic acid copolymer.
In particular, the acrylic copolymer used in the present disclosure is preferably an acrylic alkyl copolymer, an acrylates copolymer, or the like from the viewpoint of further exerting the effects of the present disclosure and improving fixability.

Examples of the acrylic resin emulsion that can be used include commercially available products such as DAITOSOL 5000STY, DAITOSOL 5000AD, DAITOSOL 5000SJ, DAITOSOL 4000SJT, DAITOSOL 5500GM, and DAITOSOL 5000PO (all available from Daito Kasei Kogyo Co., Ltd.); Yodosol GH34F and Yodosol GH800F (both available from Nouryon Japan); Vinysol 1086DB, Vinysol 1087FT, Vinysol 1089HT, Vinysol 1012JC, Vinysol 1013JH, Vinysol 2140L, Vinysol 2140LH, Vinysol 1086WP, Vinysol 1087FT, and Vinysol 1089HT (all available from Daido Chemical Corporation); Luvimer 100P (available from BASF Japan Ltd.); AMPHOMER HC (available from Nouryon Japan); and SYNTRAN EX149PE and SYNTRAN 5402 (available from INTERPOLYMER CORPORATION).

The content of the acrylic copolymers is preferably 1 to 20%, and more preferably 3 to 19% in terms of solid content with respect to the total amount of the aqueous liquid cosmetic.
When the content of the acrylic copolymer is less than 1%, a decrease in the fixability and deterioration in the vertical concentration difference due to the low viscosity are observed, and when the content is more than 20%, that is too high, the viscosity increases so much that discharge of the liquid is difficult.

The aqueous liquid cosmetic of the present disclosure preferably contains a thickener from the viewpoint of application properties, storage stability, further suppression of sedimentation of the plate-shaped pigment, and the like.
As described later, the thickener that can be used is preferably used for making the viscosity of the aqueous liquid cosmetic suitable and exerting the above-described containing effect, and it is preferable to use dextrin and/or a thickening polysaccharide.

The dextrin that can be used is generally a carbohydrate obtained by hydrolysis of starch or glycogen to low molecular weight. The dextrin may have a molecular structure in which α-glucose is polymerized by α-(1→4) or α-(1→6) glycosidic bonds.
The "dextrin" in the present disclosure means a dextrin selected from the group consisting of acyclic dextrin in which α-glucose units are bonded in a linear or branched chain, cyclic dextrin in which α-glucose units are bonded in a cyclic form, and mixtures thereof.

As the acyclic dextrin, dextrin having a dextrose equivalent (DE value) of more than 0, 1 or more, 3 or more, 5 or more, 7 or more, or 10 or more, and less than 100, 80 or less, 60 or less, 40 or less, 30 or less, 20 or less, 17 or less, or 15 or less can be used.
As the cyclic dextrin, dextrin having 5 or more, 6 or more, or 7 or more glucose units in the molecule, and 20 or less, 15 or less, or 10 or less glucose units can be used.
Examples of the cyclic dextrin include unsubstituted cyclic dextrins such as α-cyclodextrin, β-cyclodextrin and γ-cyclodextrin, and substituted cyclic dextrins such as hydroxypropylated β-cyclodextrin and maltosylcyclodextrin. Examples of the dextrin that can be used include commercially available products of 103 dextrin (available from Tokai Dextrin Co., Ltd.) and SELDEX SL-20 (available from Nippon Shoku Chemical Co., Ltd.).

Examples of the thickening polysaccharides that can be used include xanthan gum, succinoglycan, guar gum, casein, gum arabic, gelatin, amylose, agarose, agaropectin, arabinan, curdlan, callose, carboxymethyl starch, chitin, chitosan, quince seed, glucomannan, gellan gum, tamarind seed gum, diutan gum, dextran, nigeran, hyaluronic acid, pustulan, funoran, pectin, porphyran, laminaran, lichenan, carrageenan, alginic acid, tragacanth gum, alkasy gum, and locust bean gum. Among these, xanthan gum, succinoglycan, and diutan gum are preferred. Examples of the commercially available products include xanthan gum (KELZAN AR; available from Sansho Co., Ltd.), succinoglycan (Rheozan SH; available from Solvay), and diutan gum (KELCO-VIS DG-F; available from Sansho Co., Ltd.). The thickening polysaccharide may be used alone, or two or more thickening polysaccharides may be used in combination.

As will be described later, the content of each of the thickeners in the present disclosure may be any content as long as the effect of the present disclosure can be exhibited by adjusting the viscosity to a suitable viscosity range of the aqueous liquid cosmetic, and is adjusted in a suitable amount with respect to the total amount of the aqueous liquid cosmetic.

In addition to the above components, the balance of the aqueous liquid cosmetic of the present disclosure is prepared with water (purified water, ion-exchanged water, distilled water, pure water, or the like) serving as a solvent.
Furthermore, in the aqueous liquid cosmetic of the present disclosure, a humectant, an antibacterial agent, an antifoaming agent, an inorganic pigment or an organic pigment, crystalline cellulose, a viscous mineral, a surfactant, a water-soluble organic solvent, and the like can be appropriately contained within a range that does not interfere with the dispersion system and does not impair the effects of the present disclosure. Examples of the humectant that can be used include water-soluble glycols such as 1,3-butylene glycol, 1,4-butylene glycol, pentylene glycol, ethylene glycol, diethylene glycol, polyethylene glycol, propylene glycol, dipropylene glycol, and glycerin. The content of the humectants is preferably used in the range of 1 to 30%, more preferably 5 to 20%, with respect to the total amount of the composition.

Examples of the antibacterial agent that can be used include parabens, sodium dehydroacetate, phenoxyethanol, and the like. Note that, the antibacterial agent of the present disclosure includes preservatives. As parabens which are preservatives, methyl paraoxybenzoate, ethyl paraoxybenzoate, propyl paraoxybenzoate, butyl parahydroxybenzoate, isopropyl paraoxybenzoate, or the like can be used in an appropriate amount. Examples of the antifoaming agent that can be used include polydimethylsiloxane (simethicone). This polydimethylsiloxane is a silicone oil consisting of a mixture of methylated linear siloxane polymers endblocked with trimethylsiloxy units, and, as a commercially available product, KS-66 (available from Shin-Etsu Silicone Co., Ltd.) or the like can be used in an appropriate amount.

In the present disclosure, for obtaining more vivid color development, a more vivid color tone can be obtained by blending a dye within a range that does not impair the effects of the present disclosure, in addition to the coating-treated plate-shaped pigment and a chromatic pigment having a predetermined particle size.
In the present disclosure, the pH of the aqueous liquid cosmetic is preferably in a range of 6 to 9 from the viewpoint of suppressing skin irritation. The pH can be adjusted by using a pH adjuster such as 2-amino-2-methyl-1-propanol, 2-amino-2-methyl-1,3-propanediol, triethanolamine, L-arginine, aqueous ammonia, or sodium hydroxide. Desirably, the pH is adjusted by using, particularly preferably 2-amino-2-methyl-1-propanol.

In addition, when the viscosity of the aqueous liquid cosmetic in the present disclosure is too high, the aqueous liquid cosmetic is not discharged from the brush (brush head), and when the viscosity is too low, the drawn line blurs and the aqueous liquid cosmetic is difficult to use. Therefore, it is desirable that the viscosity at a shear rate of 191.5 s⁻¹ at 25°C is thus 15 mPa·s or less, preferably in a range of 2 to 10 mPa·s.
When the viscosity is within the above range, a flow rate is ensured, and a line is easily drawn without blurring, and the aqueous liquid cosmetic is easily applied.
The viscosity range can be adjusted by combining the coating-treated plate-shaped pigment, the chromatic pigment, water, and further the acrylic copolymer in suitable contents, and by adjusting suitable stirring conditions, dispersion conditions, and the like.

The aqueous liquid cosmetic of the present disclosure is prepared by mixing and dispersing the above-described components in the above-described ranges of amounts and the like with a mixing and dispersing machine, for example, a bead mill, a homomixer, a disperser, an attritor, a ball mill, a sand grinder, or the like.

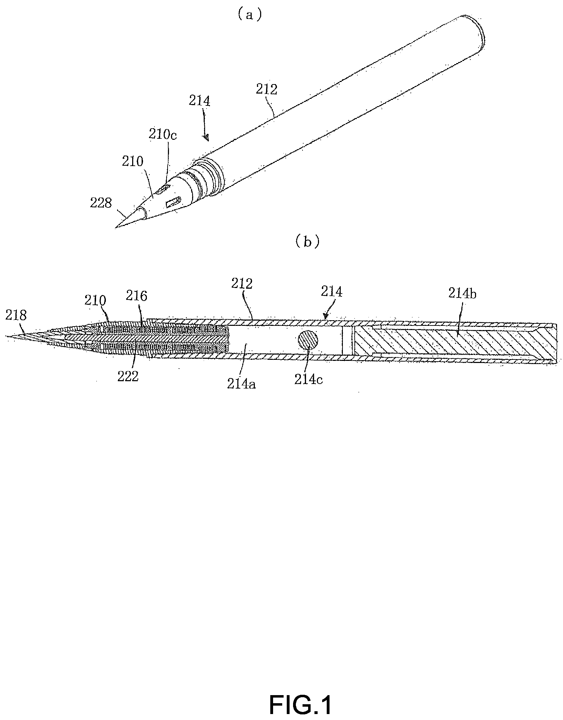

The aqueous liquid cosmetic of the present disclosure configured as described above can be suitably used by being stored in an applicator in which an application portion is formed of a brush (brush head).
In an applicator having an application portion in which fibers are solidified with a resin or the like or an application portion of a so-called pen core type in which fibers are fused to each other, the plate-shaped pigment can be slid onto an application surface by application, but an application liquid is scraped off, and as a result, the effect of increasing the sparkling effect is reduced, and the applicability is deteriorated.
Examples of the applicator that can be used include an applicator shown in FIG. 1, in which the aqueous liquid cosmetic composition having the above-described configuration is incorporated.
The liquid cosmetic applicator loaded with the aqueous liquid cosmetic of the present disclosure is, for example, a collector-type applicator, as illustrated in FIG. 1(a), having a barrel 214 in which a front barrel 210 and a barrel body 212 at the rear side of the front barrel 210 are fitted into each other. In the applicator, as illustrated in FIG. 1(b), a collector 216, which is formed in a comb-like shape in an embodiment in which a plurality of sheet portions are arranged in the axial direction, is disposed in the front portion of the barrel 214, and the liquid cosmetic composition is stored in a storage space 214a in the rear portion in the barrel 214.

The barrel body 212 at the rear portion of the barrel 214 is formed in a pipe shape that communicates with the inside and is opened in the front and rear direction. A tail plug 214b is fitted to a rear portion of the barrel body 212 that is also a rear portion of the barrel to close the rear portion of the barrel body 212. A space in the barrel 214 (also a space in the barrel body 212) sandwiched between a front end of the tail plug 214b and a rear end of the collector 216 is the storage space 214a.
In the storage space 214a, an impregnation body such as an inner cotton is not disposed, but an application liquid is directly stored, and a stirring body (e.g., ball) 214c configured to stir the application liquid is disposed.

The front barrel 210, the barrel body 212, the collector 216, and the cap may be resin molded products. A ball material made of metal or resin can be used for the stirring body 214c.
The collector 216 is covered and held by the front barrel 210 and the barrel body 212.
An application portion 218 formed of a brush body having a tapered shape protrudes from an opening at the front end portion of the front barrel 210, and a cap covering the application portion 218 is removably fitted to the front barrel 210. The front barrel 210, which has a substantially conical side surface shape, is tapered and provided with air replacement holes 210c at predetermined intervals on the peripheral surface, and the front barrel 210 is desirably formed such that the tip angle thereof is substantially equal to the tip angle of the application portion 218.

The application portion 218 is a tapered brush body (brush) made of resin fibers, natural fiber bundles, or a porous body made of resin. The rear end portion of the application portion 218 has an enlarged diameter in a flange shape. This enlarged portion is engaged with the inside of the front barrel 210 and prevents the front barrel 210 from detaching.

A bellows-like collector 216 is disposed behind the application portion 218 in the inside of the hollow tapered front barrel 210, and a core 222 penetrates through the hollow portion of the collector 216 and is disposed therein. The core 222 can be formed of a capillary member such as a resin fiber bundle, a natural fiber bundle, or a resin porous body.
In the core 222, the core 222 does not protrude from the rear end portion of the collector 216 into the storage space 214a of the barrel 214 (see FIG. 1(b)). The rear end surface of the core 222 substantially matches the rear end surface of the collector 216. By matching the core 222, the rear end of the core 222 does not protrude into the storage space 214a, and the volume in the storage space 214a can be ensured. Since the rear end of the core 222 does not protrude into the storage space 214a, when the stirring body 214c is provided in the storage space 214a, the stirring body 214c does not collide with the core 222 and does not deform the core 222 even if the stirring body 214c moves in the storage space 214a. Therefore, the coating liquid can sufficiently penetrate through the core.

The liquid cosmetic applicator of the above embodiment has been described with reference to, for example, a liquid cosmetic applicator of liquid eyeliner or liquid eyeshadow that is the aqueous liquid cosmetic of the present disclosure. However, the present disclosure is not limited thereto, and the present disclosure can also be applied to an eyebrow applicator for drawing a line on eyebrows and for drawing a line on the skin. The applicator of rotary extension type illustrated in FIG. 1 is used as a liquid pressing mechanism of the liquid cosmetic applicator of the above embodiment, but a liquid cosmetic applicator of knock extension type may be used.

The aqueous liquid cosmetic of the present disclosure configured as described above contains a small amount of a plate-shaped pigment having glittering properties enables drawing of a line having a constant hue and sparkling effect over time when the composition is loaded on an applicator having an application portion such as a brush and applied on a subjective surface such as the skin. It is presumed that that is caused by the following operational effects and the like.
According to the present disclosure, in the case of an aqueous liquid cosmetic containing at least (a) 0.01 to 10 mass% of a plate-shaped pigment having a surface coated with a compound selected from Group A, (b) at least one chromatic pigment having an average particle size of 0.01 to 1 µm as measured by dynamic light scattering, (c) 1 to 20 mass% in terms of solid content of an acrylic copolymer, and (d) water, and being loaded on an applicator having an application portion formed of a brush, chromatic pigment particles having an average particle size of 0.01 to 1 µm prevent the plate-shaped pigments from forming hard cake by entering between the plate-shaped pigments during sedimentation of the plate-shaped pigments. Further, it is presumed that by suppressing sedimentation of the plate-shaped pigment exhibiting glittering properties, a hue difference and a decrease in sparkling effects that are caused over time can be suppressed, and an aqueous liquid cosmetic can be suitably obtained for an applicator such as a direct liquid type eyeliner, an eyeshadow, and an eyebrow product for drawing a line on eyebrows, in which a drawing line having a hue and sparkling effects constant over time can be written. Therefore, the aqueous liquid cosmetic of the present disclosure has the above-described operational effects, and thus can be suitably used for eye makeup such as an eyeliner.

### Examples

Hereinafter, the present disclosure will be further described in detail with reference to examples and comparative examples. The present disclosure is not limited to the following examples and the like.

### [Examples 1 to 8 and Comparative Examples 1 to 8]

Each aqueous liquid cosmetic was prepared according to the formulation shown in Table 1 below and by mixing and dispersing using a homomixer or a disperser.

Each of the aqueous liquid cosmetics of Examples 1 to 8 and Comparative Examples 1 to 8 as obtained above was evaluated for viscosity at 25°C, sparkling effect, brightness difference, hue difference, concealing property, and fixability by the following measurement methods and evaluation methods. The pH of each of the aqueous liquid cosmetics was in a range of 6.5 to 8.5.

The results are shown in Table 1 below.

### Measuring Method of Viscosity

For each aqueous liquid cosmetic obtained, the viscosity at a predetermined shear rate (191.5 S⁻¹) at a temperature of 25°C using a cone and plate viscometer (ELD type viscometer among TV-30 viscometers, standard cone and plate, available from Tokimec) was measured.

### [Evaluation Method of Sparkling Effect, Brightness Difference, Hue Difference, Concealing Property, and Fixability]

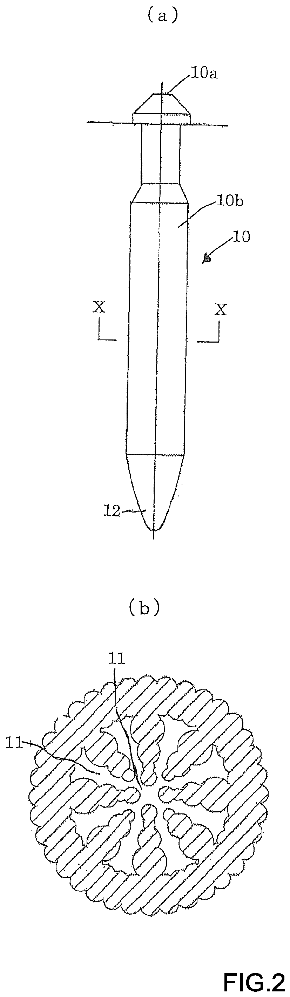

Each applicator was produced by combining the aqueous liquid cosmetic of each blending composition shown in Table 1 below with each applicator having a brush head or a pen core illustrated in FIG. 1 or FIG. 2, and evaluated for the sparkling effect, brightness difference, hue difference, concealing property, and fixability.

The brush head used in Examples of FIG. 1 is a tapered brush body formed of a bundle of polybutylene terephthalate resin fibers each having a diameter of φ 0.05 to 0.3 mm. The pen core used in Comparative Examples of FIG. 2 is obtained through a step of extrusion molding of a polyacetal resin (POM) and so forth. As illustrated in FIGS. 2(a) and 2(b), the pen core 10 is made of POM having an outer diameter of 3.0 mm and having an ink path 11 of an internal groove having a radial cross-section continuously formed in the axial direction, and the entire application portion 12 as a tip portion is cut and formed in a substantially bullet shape having a jet cone shape, and further, an aqueous liquid cosmetic having each blending composition of Table 1 below is attached to an applicator main body (not shown) having a valving mechanism, and holes (not shown) for introducing the cosmetic into the cosmetic path in the applicator main body are formed in a pen core rear end portion 10a and a pen core side surface portion 10b, and the liquid cosmetic is applied from the application portion by an application operation.

Sensory evaluation was performed on the sparkling effect, the brightness difference, the hue difference, the concealing property, and the fixability by the following evaluation method using each applicator filled with the aqueous liquid cosmetic of each of Examples and Comparative Examples having the brush head and the pen core.

### [Method of Evaluating Sparkling Effect]

Each of the aqueous liquid cosmetics was applied to the skin using the respective applicator, and subjected to a sensory evaluation in which the sparkling effect was visually evaluated in accordance with the following evaluation criteria.

Evaluation criteria:
A: The sparkling effect is high.
B: The sparkling effect is present.
C: The sparkling effect is weak.
D: There is almost no sparkling effect.

### [Method of Evaluating Brightness Difference]

After being allowed to stand for a fixed period of time (24 hours) in either an upward or downward orientation, each of the aqueous liquid cosmetics was applied to the skin using the respective applicator, and subjected to a sensory evaluation in which the brightness difference was visually observed in accordance with the following evaluation criteria.

Evaluation criteria:
A: The sparkling effect is constant.
B: The sparkling effect is almost constant.
C: The sparkling effect decreases.
D: The sparkling effect is lost.

### [Method of Evaluating Hue Difference]

Each of the aqueous liquid cosmetics was applied to the skin using the respective applicator, and subjected to a sensory evaluation in which the hue difference was visually evaluated in accordance with the following evaluation criteria.

Evaluation criteria:
A: There is no change in hue.
B: The hue is almost constant.
C: There is a slight change in hue.
D: There is a change in hue.

### [Method of Evaluating Concealing Property]

Each of the aqueous liquid cosmetics was applied to the skin using the respective applicator, and subjected to a sensory evaluation by visual observation to determine whether the skin color was concealed, in accordance with the following evaluation criteria.

Evaluation criteria:
A: The skin color is completely concealed.
B: The skin color is slightly visible through.
C: The skin color is visible through.
D: The skin color is not concealed at all.

### [Method of Evaluating Fixability]

Each of the aqueous liquid cosmetics was applied to the eye rim using the respective applicator, and after 4 hours, subjected to a sensory evaluation in which the fixability was visually evaluated in accordance with the following evaluation criteria.

Evaluation criteria for fixability:
A: There is almost no difference in the sparkling effect and color development of the drawn line from the initial stage.
B: The sparkling effect and color development of the drawn line are not much different from the initial stage.
C: The sparkling effect and color development are greatly reduced from the initial stage.
D: Almost no drawn line remains.

**[Table 1]**

| (Total amount 100 mass%) | | | | | | | | | | | | | | | | |
|---|---|---|---|---|---|---|---|---|---|---|---|---|---|---|---|---|
| | Example | | | | | | | | Comparative Example | | | | | | | |
| | 1 | 2 | 3 | 4 | 5 | 6 | 7 | 8 | 1 | 2 | 3 | 4 | 5 | 6 | 7 | 8 |
| Flaky pigment (surface-treated) A-1 *1 | 3 | | 1 | 10 | 3 | 3 | 3 | 3 | 3 | 0.005 | 15 | | 3 | 3 | 3 | 3 |
| Flaky pigment (surface-treated) A-2 *2 | | 3 | | | | | | | | | | | | | | |
| Flaky pigment A-3 *3 | | | | | | | | | | | | 3 | | | | |
| Carbon black *4 | | | | | 2 | | | | | | | | | | | |
| Red No. 226 *5 | | | | | 5 | | | | | | | | | | | |
| Iron oxide *6 | 5 | 5 | 5 | 5 | | 5 | 5 | 5 | | 5 | 5 | 5 | | | 5 | 5 |
| SiO1-2 sericite FSE *7 | | | | | | | | | | | | | | 5 | | |
| Fine particulate titanium oxide *8 | | | | | | | | | | | | | 5 | | | |
| Acrylic copolymer *9 | 7 | 7 | 7 | 7 | 7 | 4 | 15 | 7 | 7 | 7 | 7 | 7 | 7 | 7 | 0.5 | 30 |
| Dextrin (cyclodextrin) *10 | | | | | | | | 0.13 | | | | | | | | |
| Thickening polysaccharide (xanthan gum) * | 0.03 | 0.03 | 0.03 | 0.03 | 0.03 | 0.03 | 0.03 | 0.06 | 0.03 | 0.03 | 0.03 | 0.03 | 0.03 | 0.03 | 0.03 | 0.03 |
| Crystalline cellulose *12 | | | | | | | | 0.5 | | | | | | | | |
| pH Adjuster *13 | 0.3 | 0.3 | 0.3 | 0.3 | 0.2 | 0.1 | 0.6 | 0.7 | 0.3 | 0.3 | 0.3 | 0.3 | 0.6 | 0.3 | 0.3 | 1 |
| Antiseptic agent *14 | 1.9 | 1.9 | 1.9 | 1.9 | 1.9 | 1.9 | 1.9 | 1.9 | 1.9 | 1.9 | 1.9 | 1.9 | 1.9 | 1.9 | 1.9 | 1.9 |
| 1,3-Butylene glycol | 10 | 10 | 10 | 10 | 10 | 10 | 10 | 10 | 10 | 10 | 10 | 10 | 10 | 10 | 10 | 10 |
| Water (purified water) | Balance | Balance | Balance | Balance | Balance | Balance | Balance | Balance | Balance | Balance | Balance | Balance | Balance | Balance | Balance | Balance |
| Application portion | Brush | Brush | Brush | Brush | Brush | Brush | Brush | Brush | Pen core | Brush | Brush | Brush | Brush | Brush | Brush | Brush |
| Viscosity (25°C, mPa▪s) | 3.7 | 3.7 | 3.2 | 4.1 | 3.7 | 3.0 | 14.6 | 6.1 | 3.1 | 3.3 | 4.4 | 2.9 | 2.7 | 3.5 | 1.9 | 30 |
| Sparkling effect | A | A | B | A | A | A | B | A | B | D | C | B | B | A | A | D |
| Brightness difference | B | B | B | B | B | B | A | A | C | B | B | D | C | B | C | C |
| Hue difference | B | B | B | B | B | B | A | B | B | B | B | B | B | D | C | D |
| Concealing property | B | B | B | B | A | B | B | B | D | B | B | B | C | B | B | B |
| Fixability | A | A | A | A | A | B | A | A | A | A | A | A | B | A | D | D |

*1 to *14 in Table 1 are as described below.
*1: (Production Example 1) MT1030PS (available from Nippon Sheet Glass Co. Ltd.) is surface-treated with crystalline cellulose [(CEOLUS RC-591, available from Asahi Kasei Corporation)] by spraying and drying. Average particle size on smooth surface: 30 µm, surface coating ratio of 2%.
*2: (Production Example 2) MT1080PS (available from Nippon Sheet Glass Co. Ltd.) is surface-treated with crystalline cellulose [(CEOLUS RC-591, available from Asahi Kasei Corporation)] by spraying and drying. Average particle size on smooth surface: 80 µm, surface coating ratio of 2%.
*3: MT1030PS (available from Nippon Sheet Glass Co. Ltd.: surface-untreated), average particle size on a smooth surface of 10 to 70 µm.
*4: DK BLACK No. 2, average particle size: 200 nm, available from DAITO KASEI KOGYO CO., LTD.
*5: DK D&C RED N0. 30, average particle size: 300 nm, available from DAITO KASEI KOGYO CO., LTD.
*6: Red iron oxide N0. 216P, average particle size: 300 nm, available from DAITO KASEI KOGYO CO., LTD.
*7: Sericite FSE, average particle size: 10 µm, available from Sanshin Mining Ind. Co., Ltd.
*8: ST-485SA15, average particle size: 8 nm, available from Titan Kogyo, Ltd.
*9: Yodosol GH800F, available from Nouryon Japan; solid content: 45%; values in Table 1 were described in terms of solid content.
*10: Beaute by Roquette CD102, available from Roquette Freres.
*11: Keltrol, available from MP Gokyo Food & Chemical Co., Ltd.
*12: Ceolus RC-N30, available from Asahi Kasei Corporation.
*13: AMP, citric acid.
*14: Phenoxyethanol SP, available from Yokkaichi Chemical Co., Ltd.

The results in Table 1 above indicate that Examples 1 to 8, which support the present disclosure, are aqueous liquid cosmetics that have excellent sparkling effect, brightness difference, hue difference, concealing property, and fixability better than Comparative Examples 1 to 8, which fall outside the range of the present disclosure.

### Industrial Applicability

The aqueous liquid cosmetic of the present disclosure can be suitably used for a liquid eyeliner, a liquid eyeshadow, an eyebrow product for drawing a line on eyebrows, and the like.

## Claims

1. An aqueous liquid cosmetic comprising at least (a) 0.01 to 10 mass% of a plate-shaped pigment having a surface coated with a compound selected from Group A, (b) at least one chromatic pigment having an average particle size of 0.01 to 1 µm as measured by dynamic light scattering, (c) 1 to 20 mass% in terms of solid content of an acrylic copolymer, and (d) water,
the aqueous liquid cosmetic being loaded onto an applicator having an application portion formed of a brush:
Group A: cellulose, hemicellulose, lignin, chitin, and chitosan.

2. The aqueous liquid cosmetic according to claim 1, further comprising a thickener.

3. The aqueous liquid cosmetic according to claim 2, wherein the thickener is a dextrin and/or a thickening polysaccharide.

4. The aqueous liquid cosmetic according to claim 1 or 2, in which a viscosity as measured at 25°C and at a shear rate of 191.5 s⁻¹ is 15.0 mPa·s or less.
